(19) Europäisches Patentamt
European Patent Office
Office européen des brevets

(11) Veröffentlichungsnummer: **0 127 726**
**B1**

(12) # EUROPÄISCHE PATENTSCHRIFT

(45) Veröffentlichungstag der Patentschrift: **26.08.87**

(51) Int. Cl.[4]: **C 07 C 83/10**, A 61 K 31/165, A 61 K 31/16

(21) Anmeldenummer: **84100875.8**

(22) Anmeldetag: **27.01.84**

(54) **Hydroxamsäuren, ihre Herstellung und ihre Verwendung in pharmazeutischen Mitteln.**

(30) Priorität: **28.02.83 DD 248320**

(43) Veröffentlichungstag der Anmeldung: **12.12.84 Patentblatt 84/50**

(45) Bekanntmachung des Hinweises auf die Patenterteilung: **26.08.87 Patentblatt 87/35**

(84) Benannte Vertragsstaaten: **AT BE CH DE FR GB IT LI NL SE**

(56) Entgegenhaltungen:
**DD - A - 141 253**
**DD - A - 149 505**
**FR - M - 681**
**FR - M - 2 634**
**US - A - 4 136 198**

**CHEMICAL ABSTRACTS, Band 48, Nr. 22, 25. November 1954, Spalte 13658h, Columbus, Ohio, USA; T. URBANSKI et al.: "Hydroxamic acids. III. Analogs of salicylohydroxamic acid"**

**Die Akte enthält technische Angaben, die nach dem Eingang der Anmeldung eingereicht wurden und die nicht in dieser Patentschrift enthalten sind.**

(73) Patentinhaber: **VEB Fahlberg-List Magdeburg, Alt Salbke 60-63, DDR-3013 Magdeburg (DD)**

(72) Erfinder: **Schewe, Tankred, Dr., Karl-Maron-Strasse 32, DDR-1140 Berlin (DD)**
Erfinder: **Strumpf, Thomas, Dr., Toni-Stemmler-Strasse 16, DDR-1500 Potsdam (DD)**
Erfinder: **Slapke, Jürgen, Dr., Kollwitzstrasse 41, DDR-1055 Berlin (DD)**
Erfinder: **Kühn, Hartmut, Dr., Pettenkoferstrasse 39, DDR-1035 Berlin (DD)**
Erfinder: **Rapoport, Samuel Mitja, Prof. Dr. Dr., Kuckhoffstrasse 45, DDR-1110 Berlin (DD)**
Erfinder: **Zanke, Dieter, Dr., Hans-Marchwitza-Ring 29, DDR-1502 Potsdam-Babelsberg (DD)**
Erfinder: **Lyr, Horst, Prof. Dr., Georg-Herwegh-Strasse 8, DDR-1300 Eberswalde (DD)**
Erfinder: **Grupe, Renate, Dr., Leninallee 218, DDR-1156 Berlin (DD)**

(74) Vertreter: **Patentanwälte Beetz sen. - Beetz jun. Timpe - Siegfried - Schmitt-Fumian, Steinsdorfstrasse 10, D-8000 München 22 (DE)**

EP 0 127 726 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue Hydroxamsäuren, Verfahren zu ihrer Herstellung sowie ihre Verwendung in pharmazeutischen Mitteln.

Die Verbindungen besitzen wertvolle pharmakologische, insbesondere antiasthmatische, antianaphylaktische antiphlogistische und antithrombotische Eigenschaften und sind in der Human- und Veterinärmedizin für die Anwendung in der Therapie des Asthma bronchiale und anderer allergischer Erkrankungen, von entzündlichen Prozessen verschiedener Art sowie von Thrombosen geeignet.

Die Synthese von Hydroxamsäuren aus Carbonsäurederivaten und Hydroxylamin ist in der Literatur hinreichend beschrieben (L. Bauer und O. Exner, Angew. Chem. 86 (1974) 419). Dabei können allerdings die langen Reaktionszeiten und erzielten Ausbeuten häufig nicht befriedigen.

Die pharmakologische Wirkung von Salicylhydroxamsäure wurde 1976 erstmals beschrieben (Opperdoes et al., Exp. Parasitol. 40 (1976) 198). Salicylhydroxamsäure zeigt trypanocide Wirkungen (z.B. gegen die Erreger der afrikanischen Schlafkrankheit, der südamerikanischen Chagas-Krankheit sowie der Nagana und anderer tropischer Viehseuchen). 4-Aminosalicylhydroxamsäure besitzt antimykotische Eigenschaften (GB-A-744 307). Einige ω-(2′-Naphthoxy)-alkylhydroxamsäuren wurden für den Einsatz als Fungizide und Bakterizide (DD-A-141 253) sowie Ureasehemmstoffe (DD-A-149 505) in der Landwirtschaft bereits vorgeschlagen. Auch die Verwendung von Hydroxynaphthylhydroxamsäuren in fungiciden und baktericiden Mitteln (DD-A-140 836) ist bekannt. Über eine Verwendung dieser Verbindungen als Pharmaka in der Human- und Veterinärmedizin wurden jedoch keine Angaben gefunden.

Aus US-A-4 136 198 sind pharmazeutische Zusammensetzungen mit Wirksamkeit gegen Trypanosomiase bekannt, die Salicylhydroxamsäure oder Halogenbenzohydroxamsäuren oder deren $C_{1-4}$-Alkylester als Eisenchelatbildner enthalten und den aeroben und anaeroben Glucoseabbau inhibieren.

FR-M-2634 beschreibt o-Aminobenzohydroxamsäure-monoethanolaminester als pharamzeutischen Wirkstoff mit hypnotischen und sedativen Eigenschaften.

Chemical Abstracts 48 (1954) 13658 h sind Methylsalicylhydroxamsäuren, 2-Hydroxy-3-naphthohydroxamsäure, 1-Hydroxy-2-naphthohydroxamsäure und 8-Hydroxy-7-chinolinhydroxamsäure als gegen Mycobakterien wirksame Verbindungen zu entnehmen.

Der Erfindung liegt die Aufgabe zugrunde, Hydroxamsäuren mit wertvollen pharmakologischen und insbesondere antiasthmatischen Eigenschaften, ein Verfahren zu ihrer Herstellung sowie pharmazeutische Mittel anzugeben. Die Aufgabe wird anspruchsgemäss gelöst.

Die erfindungsgemässen neuen Hydroxamsäuren besitzen die allgemeine Formel I

R–CO–NH–OH (I),

in der R bedeutet:
- 2-Hydroxy-1-naphthyl,
- 1′-Naphthoxy)-phenylmethyl oder
- substituiertes 2′-Naphthoxymethyl der Formel II

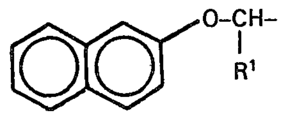
(II)

mit $R^1$ = p-Chlorphenyl oder p-Tolyl.

Das Verfahren gemäss der Erfindung zur Herstellung der Hydroxamsäuren der Formel I mit R = 2′-Naphthoxymethyl der Formel II ist gekennzeichnet durch an sich bekannte Umsetzung von Estern oder Amiden von Carbonsäuren der allgemeinen Formel IV

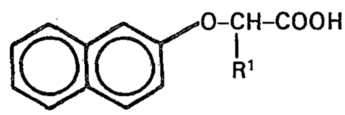
(IV)

mit $R^1$ = p-Chlorphenyl oder p-Tolyl mit Hydroxylamin oder einem Salz davon in einem basischen Lösungsmittelmedium bei einer Temperatur von 20 bis 100 °C bzw. der Siedetemperatur des Lösungsmittels und ggfs. Überführung der erhaltenen Hydroxamsäuren der Formel I mit einer anorganischen Base oder einem Metallsalz in entsprechende Salze.

Die erfindungsgemässen pharmazeutischen Mittel auf der Basis von Hydroxamsäuren sind gekennzeichnet durch Hydroxamsäuren der allgemeinen Formel I′

R–CO–NH–OH (I′)

als Wirkstoffe, wobei R bedeutet:
- 2-Hydroxy-1-napthyl, oder
- 1′-Naphthoxyalkyl der Formel IIa

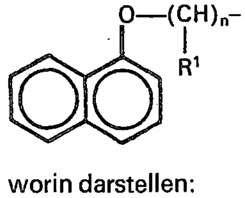
(IIa),

worin darstellen:

n 1 bis 10 und

$R^1$ für n = 1 bis 10: Wasserstoff und ausserdem für

n = 1: Geradkettiges oder verzweigtes $C_{1-10}$-Alkyl oder Phenyl, oder wobei R bedeutet: 2′-Naphthoxyalkyl der Formel IIb

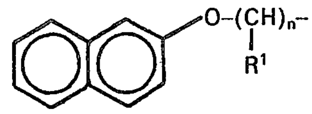
(IIb),

worin darstellen:

n 1 bis 10 und

$R^1$ für n = 1 bis 10: Wasserstoff und ausserdem für

n = 1: Geradkettiges oder verzweigtes $C_{1-10}$-Alkyl, Phenyl oder substituiertes Phenyl.

Gemäss der Erfindung werden ferner Hydroxamsäuren der allgemeinen Formel I'',

R–CO–NH–OH (I''),

in der R 1-Hydroxy-2-naphthyl, 2-Hydroxy-3-naphthyl oder 2-Amonophenyl bedeutet, zur Herstellung von pharmazeutischen Mitteln mit Lipoxygenase-Hemmwirkung zur Anwendung als Antiasthmatica, Antiallergica, Antiphlogistica, Bronchodilatantien, Antirheumatica und Antithrombotica verwendet.

Die Hydroxamsäuren der allgemeinen Formel I besitzen pharmakologisch wertvolle, insbesondere antiasthmatische, antiallergische, entzündungshemmende und antithrombotische Eigenschaften und können daher als entsprechende Wirkstoffe in pharmazeutischen Mitteln verwendet werden.

Das Verfahren gemäss der Erfindung zur Herstellung von 2'-Naphthoxymethylhydroxamsäuren wird mit Reaktionszeiten zwischen 20 min. und 15 h durchgeführt.

Als geeignetes Lösungsmittel werden unter den Reaktionsbedingungen inerte organische Lösungsmittel verwendet, wie beispielsweise niedere Alkohole, Acetonitril und Chloroform. Weiterhin können Aceton, Diethylether, Di-n-butylether, Essigsäureethylester, 1,2-Dichlorethan, Dimethylformamid, Dimethylsulfoxid, 1,4-Dioxan, Methylenchlorid, Nitromethan, Petrolether, Tetrachlorethylen, Tetrachlorkohlenstoff und Trichlorethylen, sowie Benzol, Nitrobenzol, Pyridin und Toluol verwendet werden.

Die Auswahl des jeweils vorteilhaften Lösungsmittels ist auch von der Art der jeweils eingesetzten Ausgangsstoffe abhängig. Zum Beispiel wird die Umsetzung eines niedrigen Alkylesters oder Amids einer Säure der Formel IV in einem niederen Alkohol, vorzugsweise Methanol, ausgeführt. Bei der Umsetzung eines aktivierten Esters der Formel IV wird beispielsweise Acetonitril, Chloroform, Dimethylformamid oder Essigsäureethylester als Lösungsmittel verwendet. Die genannten Amide und Ester von Carbonsäuren der allgemeinen Formel IV werden mit Hydroxylamin oder einem Salz davon in stöchiometrischen Mengen oder mit einem Hydroxylaminüberschuss, vorzugsweise einem 2- bis 10fachen Überschuss, umgesetzt. Bei der Umsetzung der genannten Carbonsäurederivate mit Hydroxylaminsalzen ist eine im Vergleich mit dem Hydroxylamin stärkere Base im Überschuss zuzugeben.

Als Amide von Säuren der Formel IV verwendet man vorzugsweise die am Amidstickstoff unsubstituierten Verbindungen. Als Ester von Säuren der Formel IV werden vorzugsweise niedere Alkylester verwendet. Als aktivierte Ester können z.B. die p-Nitrobenzyl-, die Ethoxycarbonylmethyl-, die Methoxymethyl- und die p-Nitrothiophenylester und vorzugsweise die Cyanomethyl- oder die p-Nitrophenylester der Carbonsäuren der Formel IV verwendet werden.

Bei der Umsetzung von niederen Alkylestern oder Amiden der Carbonsäuren der Formel IV wird eine dem Hydroxylaminsalz äquivalente Menge, vorzugsweise jedoch ein 2- bis 4facher Überschuss, einer Base, wie z.B. Natriumalkoholat oder alkoholische Natron- oder Kalilauge, hinzugefügt.

Werden aktivierte Ester, wie beispielsweise der Cyanomethylester oder der p-Nitrophenylester, einer Carbonsäure der Formel IV umgesetzt, so wird eine dem Hydroxylaminhydrochlorid mindestens doppelt äquivalente Menge einer tertiären Base, wie z.B. Triethylamin oder Pyridin, zugegeben.

Niedrige Alkylester werden beispielsweise durch Umsetzen von Alkalinaphtholaten mit Halogencarbonsäureestern in niederen Alkoholen oder Aromaten direkt oder durch Veresterung der entsprechenden Säuren der Formel IV oder durch Alkoholyse der entsprechenden Nitrile hergestellt. Aus den erhaltenen niederen Alkylestern werden nach Umsetzung mit Ammoniak in bekannter Weise die entsprechenden Amide erhalten.

Die erfindungsgemässen Hydroxamsäuren können gewünschtenfalls in bekannter Weise durch Umsetzung mit einer anorganischen Base oder einem Metallsalz in ihre Salze überführt werden.

Zur Salzbildung können Alkalihydroxide, z.B. Natriumhydroxid oder Kaliumhydroxid, oder Erdalkalihydroxide, z.B. Calciumhydroxid, oder entsprechende Alkali- und Erdalkalihalogenide verwendet werden.

Überraschenderweise wurde gefunden, dass sich die Verbindungen der allgemeinen Formel I und ihre Salze durch günstige pharmakologische Eigenschaften auszeichnen. Sie können in der Human- und Veterinärmedizin zur Therapie des Asthma bronchiale und anderer allergischer Erkrankungen, von Entzündungen und von Thrombosen eingesetzt werden.

Bevorzugt werden pharmakologisch gut verträgliche Salze der Verbindungen der Formel I verwendet. Hierunter sind insbesondere Salze mit solchen Basen zu verstehen, deren Kationen bei den in Frage kommenden Dosierungen entweder keine oder eine erwünschte eigene pharmakologische Wirkung zeigen. Ferner ist es von Vorteil, wenn die als Wirkstoff zu verwendenden Salze gut kristallisierbar und nicht oder nur schwach hygroskopisch sind. Zur Salzbildung mit Verbindungen der allgemeinen Formel I können zum Beispiel anorganische Basen verwendet werden, wie z.B. wässerige oder wässerig/alkoholische Lösungen von Alkali- oder Erdalkalihydroxiden.

Die Verbindungen der allgemeinen Formel I besitzen pharmakologisch wertvolle, insbesondere antiasthmatische, antiallergische, entzündungshemmende und antithrombotische Eigenschaften.

Sie zeigen in tierexperimentellen Untersuchungen am isolierten Lungenstreifen von Meerschweinchen einen ausgeprägten antiasthmatischen Effekt. Die Prüfung auf die genannte pharmakologische Eigenschaft erfolgte nach prinzipiell aus der Literatur bekannten Messmethoden, die in modifizierter Form zur Anwendung kamen (J.M. Drazen et al., J. Clin. Invest. 63 (1979) 1; M.W. Schneider und J.M. Drazen, Amer. Rev. Resp. Dis. 121 (1980) 835; S.S. Yen und W. Kreutner, Agents Actions 10 (1980) 274; S.S.Yen, Prostaglandins 22 (1981) 183).

Die Verbindungen der Formel I bewirken eine signifikante Hemmung der Arachidonsäure-induzierten Bronchokonstriktion ($1{,}5 \cdot 10^{-5}$ M Arachidonsäure) an Meerschweinchenlungenpräparaten (vgl. Beispiel 6). Auch bei Zugabe eines Cyclooxygenasehemmstoffs zum Messsystem war dieser Effekt unverändert, z.T. stärker, nachweisbar. Dieses experimentelle Ergebnis lässt zweifelsfrei die Schlussfolgerung zu, dass es sich bei den Verbindungen der Formel I um Hemmstoffe des Lipoxygenasewegs des Arachidonsäuremetabolismus handelt, und der oben beschriebene pharmakologische Effekt auf diesem Wirkungsmechanismus beruht.

Diese Schlussfolgerung wird auch auf direktem Wege durch Untersuchung an einer hoch gereinigten Lipoxygenase aus Kaninchenretikulocyten bestätigt (vgl. Beispiel 7). Die Lipoxygenase aus Kaninchenretikulocyten wurde nach einem aus der Literatur bekannten Verfahren (S.M. Rapoport et al., Methods in Enzymology 71 (1981) 430) in elektrophoretisch und immunologisch reiner Form erhalten. Die Bestimmung der Lipoxygenaseaktivität erfolgte bei 25 °C über die polarographische Messung des Sauerstoffverbrauchs mittels Clark-Elektrode in einem standardisierten System.

Die Verbindungen der Formel I zeigten in einer Endkonzentration von $10^{-4}$ M eine 100%ige Hemmung. Durch Variation der Substanzkonzentrationen konnten die Titrationskurven der Hemmung und daraus die Hemmungskonzentrationen (I-Werte) der Verbindungen ermittelt werden.

Dabei ergaben sich beispielsweise für die 2-Hydroxy-1-naphthylhydroxamsäure ein $I_{50}$-Wert von 1,6 µM, für die 2-(2'-Naphthoxy)-acethydroxamsäure ein $I_{50}$-Wert von 2,5 µM und für die 2(2'-Naphthoxy)-2-n-nonylacethydroxamsäure ein $I_{50}$-Wert von 1,5 µM.

Damit übertreffen diese Verbindungen deutlich das aus dem Stand der Technik bekannte 3-Amino-N-(3'-trifluormethylphenyl)-pyrazolin-(2), [an Wirksamkeit] das in der neueren Literatur bekannteste lipoxygenasehemmende Antiasthmatikum, [–] das unter gleichen Testbedingungen wie oben einen $I_{50}$-Wert von 20 µM besitzt.

Da für die neuen Arzneimittel die Hemmung der Lipoxygenase als molekularer Angriffsort identifiziert wurde, wurde auch ihr Einfluss auf die Thrombocytenaggregation untersucht. Die essentielle Rolle des Lipoxygenasewegs für die Irreversibilität der Thrombocytenaggregation ist heute bekannt (C.E. Dutilh et al., Prostaglandins and Medicine 6 (1981) 111). Der Irreversibilität der Thrombocytenaggregation wird bei der Pathogenese thrombotischer Erkrankungen eine Schlüsselrolle zugeschrieben. Daher ist der Befund von ausserordentlicher Bedeutung, dass die Verbindungen der Formel I die Thrombocytenaggregation je nach Versuchsbedingungen entweder vollständig hemmen oder reversibel machen (vgl. Beispiel 8). In den Experimenten wurde die Thrombocytenaggregation entweder durch Arachidonsäure oder durch den Plättchenaktivierungsfaktor (PAF-Acether) ausgelöst.

Die Hemmung der Thrombocytenaggregation ist ebenfalls auf die Hemmung der Lipoxygenase in diesen Zellen zurückzuführen. So wurde gezeigt, dass die Arachidonat-Lipoxygenaseaktivität eines mikrosomenfreien Lysats aus menschlichen Thrombocytenkonzentraten gehemmt wird. Daneben wurde nachgewiesen, dass auch eine Reihe weiterer Lipoxygenasen durch die erfindungsgemässen Hydroxamsäuren gehemmt werden. Am unempfindlichsten reagierten die Lipoxygenase aus Sojabohnen, die durch 0,4 mM 2-Hydroxy-1-naphthylhydroxamsäure zu 54% gehemmt wurde. Dagegen ergaben sich für die Lipoxygenase aus Erbsen, die in ihren Eigenschaften den tierischen Lipoxygenasen näher kommt, folgende $I_{50}$-Werte: 2-Hydroxy-1-naphthylhydroxamsäure 2 µM, 2-(2'-Naphthoxy)-acethydroxamsäure 2 µM. Weiterhin wurde gezeigt, dass die Bildung von Lipoxygenaseprodukten der Arachidonsäure in in vitro kultivierten Aortaendothelzellen des Kalbs durch 0,1 mM 2-Hydroxy-1-naphthylhydroxamsäure verhindert wird. Diese Ergebnisse erlauben die Schlussfolgerung, dass die hier beschriebenen Hydroxamsäuren universelle Hemmstoffe von Lipoxygenasen sind, worauf die nachgewiesenen pharmakologischen Wirkungen zurückzuführen sind.

Die entzündungshemmende Wirkung der Verbindungen der Formel I drückt sich u.a. in einer Hemmung des Carrageeninödems der Rattenpfote aus (vgl. Beispiel 9).

Die antiallergische (antianaphylaktische) Wirkung der Verbindungen der Formel I zeigt sich im Test der passiven cutanen Anaphylaxie. Nach intracutaner Applikation hemmten einige Verbindungen zu ca. 40%, d.h. in dem Masse, wie es in der Literatur für einen selektiven Lipoxygenasehemmstoff bekannt ist (J. Morley et al., Agents Actions 11 (1981) 585).

Die genannten Experimente belegen an geeigneten biologischen Modellen den antiasthmatischen, antiallergischen, antithrombotischen sowie den antiinflammatorischen Effekt.

Als Indikationsgebiete in der Human- und Veterinärmedizin können beispielsweise genannt werden:

1. Alle Formen des Asthma bronchiale einschliesslich des infektbedingtenAsthma bronchiale (intrinsic asthma), des exogen-allergischen Asthma bronchiale (extrinsic asthma) vom Typ I, II und IV nach Coombs und Gell (R.R.A. Coombs und P.G.H. Gell: The classification of allergic reactions responsible for clinical hypersensitivity and disease. In: Clinical aspects of immunology,

ed. by P.G.H. Gell and R.R.A. Coombs, S. 575, Blackwell Scientific Publications, Oxford, 1968), des Analgeticainduzierten Asthma bronchiale (aspirin-induced asthma), des belastungsinduzierten Asthma bronchiale (exercise-induced asthma), des Kälteasthma, des irritativ bedingten Asthma bronchiale und des psychogen ausgelösten Asthma bronchiale.

2. Asthmoide Bronchitis und obstruktives Lungenemphysem sowie alle bronchokonstriktorischen Zustände, die als Begleitsymptom anderer Erkrankungen oder Nebenwirkung medizinischer Massnahmen, z.B. Narkosekomplikationen oder bronchospastische Reaktionen nach Applikation beta-adrenerger Blockersubstanzen, auftreten.

3. Allergische Erkrankungen im weiteren Sinne, insbesondere:

- atopische Dermatitis,
- allergische Rhinitis (saisonale Rhinitis, perenniale Rhinitis, aber auch vasomotorische Rhinitis),
- Urticaria,
- Angioödem,
- Kontaktdermatitis (Kontaktekzem),
- allergische Erkrankungen des Gastrointestinaltraktes.

4. Alle Formen der Thrombose, sowohl zur Behandlung bestehender Thrombose (Thrombophlebitis) als auch

- chronisch-ischämischer Herzkrankheit,
- Nachbehandlung bei Myocardinfarkt,
- chronisch rezidivierende Thrombose,
- chronische Thrombophlebitis.

5. Der Einsatz als entzündungshemmende Arzneimittel auf der Basis nichtsteroider Antiphlogistica, wobei die Verbindungen der Formel I vor allem bei solchen entzündlichen Prozessen indiziert sind, bei denen die herkömmlichen Antiphlogistica (z.B. Acetylsalicylsäure, Salicylat u.a.), die einen Angriffspunkt ausserhalb der Lipoxygenase aufweisen, ungenügende therapeutische Effekte zeigen, insbesondere bei purulenten Entzündungen und rheumatischen Erkrankungen.

Die Verbindungen der allgemeinen Formel I eignen sich als Wirkstoff für oral, rectal, parenteral oder percutan sowie als Aerosol anwendbare Arzneimittel zur Behandlung von verschiedenen Formen des Asthma bronchiale sowie von Thrombosen rheumatischen, arthritischen und anderen entzündlichen Krankheiten.

Aufgrund ihrer besonders günstigen pharmakologischen Eigenschaften sind zu nennen:

2-Amino-benzhydroxamsäure
2-Hydroxy-1-naphthylhydroxamsäure
1-Hydroxy-2-naphthylhydroxamsäure
2-Hydroxy-3-naphthylhydroxamsäure
2-(2'-Naphthoxy)-acethydroxamsäure
2-(2'-Naphthoxy)-2-methyl-acethydroxamsäure
2-(2'-Naphthoxy)-2-ethyl-acethydroxamsäure
2-(2'-Naphthoxy)-2-n-propyl-acethydroxamsäure
2-(2'-Naphthoxy)-2-i-propyl-acethydroxamsäure
2-(2'-Naphthoxy)-2-n-butyl-acethydroxamsäure
2-(2'-Naphthoxy)-2-n-pentyl-acethydroxamsäure
2-(2'-Naphthoxy)-2-n-hexyl-acethydroxamsäure
2-(2'-Naphthoxy)-2-n-nonyl-acethydroxamsäure
2-(2'-Naphthoxy)-2-phenyl-acethydroxamsäure
2-(2'-Naphthoxy)-2-p-tolyl-acethydroxamsäure
2-(2'-Naphthoxy)-2-p-chlorphenyl-acethydroxamsäure
3-(2'-Naphthoxy)-propylhydroxamsäure
4-(2'-Naphthoxy)-butylhydroxamsäure
5-(2'-Naphthoxy)-valerylhydroxamsäure
7-(2'-Naphthoxy)-heptylhydroxamsäure
8-(2'-Naphthoxy)-octylhydroxamsäure
11-(2'-Naphthoxy)-undecylhydroxamsäure
2-(1'-Naphthoxy)-acethydroxamsäure
2-(1'-Naphthoxy)-2-methyl-acethydroxamsäure
2-(1'-Naphthoxy)2-ethyl-acethydroxamsäure.

Die pharmazeutischen Artikel gemäss der Erfindung enthalten neben nichttoxischen, inerten pharmazeutisch geeigneten Trägerstoffen einen oder mehrere erfindungsgemässe Wirkstoffe.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungsformen seien Tabletten, Dragees, Kapseln, Pillen, Granulate, Suppositorien, Lösungen, Suspensionen und Emulsionen, Pasten, Salben, Gele, Cremes, Lotionen, Puder, Sprays und Aerosole genannt.

Tabletten, Dragees, Kapseln, Pillen und Granulate können den oder die Wirkstoffe neben den üblichen Trägerstoffen enthalten, wie a) Füll- und Streckmittel, z.B. Stärken, Milchzucker, Rohrzukker, Glucose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumbicarbonat, e) Lösungsverzögerer, z.B. Paraffin, f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit, und i) Gleitmittel, z.B. Talkum, Calcium- und Magnesiumstearat und feste Polyethylenglycole oder Gemische der unter a) bis i) aufgeführten Stoffe.

Die Tabletten, Dragees, Kapseln, Pillen und Granulate können mit den üblichen, gegebenenfalls Opalisierungsmittel enthaltenden Überzügen versehen und auch so zusammengesetzt sein, dass sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der eben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B.

Polyethylenglycole, Fette, z.B. Kakaobutter und höhere Ester (z.B. $C_{14}$-Alkohol mit $C_{16}$-Fettsäure) oder Gemische dieser Stoffe.

Salben, Pasten, Cremes und Gelee können neben dem oder den Wirkstoffen die üblichen Trägerstoffe enthalten, z.B. tierische und pflanzliche Fette, Wachse, Paraffine, Stärke, Tragant, Cellulosederivate, Polyethylenglycole, Silicone, Bentonite, Talkum, Kieselsäure und Zinkoxid oder Gemische dieser Stoffe.

Sprays und Puder können neben dem oder den Wirkstoffen die üblichen Trägerstoffe enthalten, z.B. Milchzucker, Talkum, Kieselsäure, Aluminiumhydroxid, Calciumsilicat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe, wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglycol, 1,3-Butylenglycol, Dimethylformamid, Öle, insbesondere Baumwollsaatöl, Erdnussöl, Cashewnussöl, Maiskeimöl, Olivenöl, Ricinusöl und Sesamöl, Glycerin, Glycerinformal, Tetrahydrofurfurylalkohol, Polyethylenglycole und Fettsäureester des Sorbitans oder Gemische dieser Stoffe, enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe, wie flüssige Verdünnungsmittel, z.B. Wasser, Ethylalkohol, Propylenglycol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar oder Tragant oder Gemische dieser Stoffe, enthalten.

Die genannten Formulierungsformen können auch Färbemittel, Konservierungsstoffe sowie geruchs- und geschmacksverbessernde Zusätze, z.B. Pfefferminzöl und Eucalyptusöl, und Süssmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Masse-% der Gesamtmischung liegen.

Die oben aufgeführten pharmazeutischen Mittel können ausser den erfindungsgemässen Wirkstoffen auch weitere pharmazeutische Wirkstoffe enthalten.

Ihre Herstellung erfolgt in üblicher Weise nach bekannten Methoden, z.B. durch Mischen der Wirkstoffe mit den Trägerstoffen.

Zur vorliegenden Erfindung gehört auch die Verwendung der erfindungsgemässen Wirkstoffe der Formel I" zur Herstellung pharmazeutischer Mittel, die einen oder mehrere Wirkstoffe enthalten, in der Human- und Veterinärmedizin zur Verhütung, Besserung und/oder Heilung der oben angeführten Krankheiten.

Die Wirkstoffe oder die pharmazeutischen Mittel können lokal, oral, parenteral, intraperitoneal und/oder rectal, vorzugsweise oral, und insbesondere als Aerosol appliziert werden.

Im allgemeinen hat es sich als vorteilhaft erwiesen, den oder die erfindungsgemässen Wirkstoffe in Gesamtmengen von etwa 10 bis etwa 300 und vorzugsweise 50 bis 200 mg/kg Körpergewicht je 24 Stunden, gegebenenfalls in Form mehrerer Einzelgaben, zur Erzielung der gewünschten Ergebnisse zu verabreichen.

Es kann jedoch erforderlich sein, von den genannten Dosierungen abzuweichen, und zwar in Abhängigkeit von der Art und dem Körpergewicht des zu behandelnden Objektes, der Art und der Schwere der Erkrankung, der Art der galenischen Formulierung und der Applikation des Arzneimittels sowie dem Zeitraum bzw. Intervall, innerhalb dessen die Verabreichung erfolgt. So kann es in einigen Fällen ausreichend sein, mit weniger als der oben genannten Wirkstoffmenge auszukommen, während in anderen Fällen die oben angeführte Menge an Wirkstoff überschritten werden muss.

Die nachfolgenden Beispiele erläutern die Erfindung.

Beispiele erfindungsgemässer 2'-Naphthoxyalkylhydroxamsäuren sind in Tabelle 1 aufgeführt.

Tabelle 1

2'-Naphthoxyalkylhydroxamsäuren

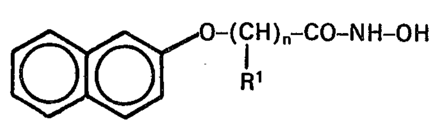

| $R^1$ | n | F. (°C) |
|---|---|---|
| $-n-C_3H_7$ | 1 | krist.-amorph |
| $-n-C_4H_9$ | 1 | 141 (Z.) |
| $-i-C_4H_9$ | 1 | krist.-amorph |
| $-n-C_5H_{11}$ | 1 | 144 (Z.) |
| $-n-C_7H_{15}$ | 1 | 152 (Z.) |
| $-n-C_8H_{17}$ | 1 | 158 (Z.) |
| $-p-C_6H_4-CH_3$ | 1 | 117–8 (Z.) |
| $-p-C_6H_4-Cl$ | 1 | 133–4 (Z.) |
| -H | 4 | 130–1 (Z.) |
| -H | 5 | 127 (Z.) |
| -H | 7 | 125–6 (Z.) |
| -H | 9 | 122–3 (Z.) |

Beispiel 1

Herstellung von 5-(2'-Naphthoxy)-valerylhydroxamsäure

Zu einer aus 4,6 g Natrium und 6,9 g Hydroxylaminhydrochlorid bereiteten absoluten methanolischen Lösung tropft man unter Rühren 25,9 g 5-(2'-Naphthoxy)-valeriansäuremethylester (aus 5-(2'-Naphthoxy)-valeriansäure und Methanol,

Kp. 171–2 °C/2,4 mbar) gelöst in 30 ml absolutem Methanol zu und erhitzt anschliessend 60 Minuten unter Rückfluss. Man kühlt ab und engt die Lösung im Vakuum bei Raumtemperatur zur Trockne ein. Den Rückstand versetzt man mit 500 ml Wasser und 100 ml Ether, rührt 20 Minuten, trennt die wässerige Lösung ab und säuert sie mit 6 n Salzsäure an. Die Suspension wird dreimal mit je 100 ml gesättigter Natriumchloridlösung gewaschen, über Natriumsulfat getrocknet und das Lösungsmittel im Vakuum entfernt. Man erhält nach Umkristallisieren aus $H_2O$/Methanol 15,8 g 5-(2'-Naphthoxy)-valerylhydroxamsäure, F. 130–1 °C (Z.).

Beispiel 2

Herstellung von 2-(2'-Naphthoxy)-2-n-octyl-acethydroxamsäure

a) 2- (2'-Naphthoxy) -2-n-octyl -essigsäureethylester 2,3 g Natrium werden in 100 ml absolutem Alkohol gelöst, mit 14,4 g 2-Naphthol versetzt und im Vakuum bis zur Gewichtskonstanz erhitzt. Dann gibt man 150 ml absolutes Toluol und anschliessend noch 37 g 2-Bromdecancarbonsäureethylester zu.

Das Reaktionsgemisch wird bei 150–160 °C bis zur neutralen Reaktion am Rückfluss erhitzt und mit Wasser versetzt, worauf die organische Phase mit Ether extrahiert wird. Die etherische Lösung wird mit Natriumhydrogencarbonatlösung gewaschen, mit Magnesiumsulfat getrocknet und das Lösungsmittel abgezogen. Man erhält nach Fraktionierung des Rückstandes 26,8 g 2-(2'-Naphthoxy)-2-n-octyl-essigsäureethylester als schwachgelbliches Öl. Kp. 186–7 °C 0,7 mbar.

b) 2-(2'- Naphthoxy) -2-n-octyl -acethydroxamsäure

Zu einer aus 2,3 g Natrium und 6,9 g Hydroxylaminhydrochlorid bereiteten absoluten alkoholischen Lösung gibt man 3,7 g 2-(2'-Naphthoxy)-2-n-octyl-essigsäureethylester und rührt bei 40 °C 8 Stunden. Anschliessend wird noch eine Stunde auf dem siedenden Wasserbad erhitzt und der Alkohol bei Raumtemperatur im Vakuum entfernt. Der Rückstand wird eine Woche stehengelassen und dann abgesaugt. Man wäscht ihn mit Wasser und trocknet die zurückbleibende Hydroxamsäure. Es werden 2,9 g 2-(2'-Naphthoxy)-2-n-octyl-acethydroxamsäure erhalten. F. 158 °C (Z.) ($H_2O$/EtOH).

Beispiel 3

Herstellung von 10-(2'-Naphthoxy)-decylhydroxamsäure

a) 10(2'- Naphthoxy)-decansäure-cyanomethylester

Eine Mischung von 8,5 g 10-(2'-Naphthoxy)-decansäure (F. 144 °C (Ether/Petrolether) ), 2,3 g Chloracetonitril und 3 g Triethylamin in 50 ml Essigsäureethylester wird 10 Stunden bei 60 °C gerührt, abgekühlt und vom abgeschiedenen Triethylaminhydrochlorid abgetrennt. Das Filtrat wird mit 10 ml 1 n Salzsäure, zweimal mit je 15 ml Natriumhydrogencarbonatlösung sowie 15 ml Wasser gewaschen und über Natriumsulfat getrocknet; das Lösungsmittel wird bei 30 °C im Vakuum entfernt. Man erhält 3,8 g 10-(2'-Naphthoxy)-decansäure-cyanomethylester. F. 121–2 °C (MeOH).

b) 10-(2'-Naphthoxy)-decylhydroxamsäure

Eine Mischung von 3,5 g 10-(2'-Naphthoxy)-decansäurecyanomethylester und 0,7 g Hydroxylaminhydrochlorid in 30 ml Acetonitril wird mit 2 Tropfen Eisessig sowie 1,05 g Triethylamin versetzt. Die Mischung wird 30 Minuten bei Raumtemperatur gerührt, nochmals mit 0,35 g Hydroxylaminhydrochlorid und 0,5 g Triethylamin versetzt und weitere 12 Stunden bei Raumtemperatur gerührt. Anschliessend wird die Mischung im Wasserstrahlpumpenvakuum bei 40 °C eingeengt, der Rückstand mit 30 ml Wasser und zweimal 50 ml Essigsäureethylester versetzt, die organische Phase abgetrennt und das Lösungsmittel im Vakuum entfernt. Den Rückstand löst man in 100 ml Ether und extrahiert die Etherlösung mit 10 ml 1 n Natronlauge, wobei sich das 10-(2'-Naphthoxy)-decanohydroxamsäure-Na-Salz kristallin abscheidet. Die Kristalle werden abfiltriert und in 30 ml absolutem Ether suspendiert; anschliessend wird die Suspension unter Kühlung mit getrocknetem Chlorwasserstoff versetzt. Nach mehrmaligem Schütteln wird die etherische Lösung filtriert und der Ether im Vakuum vertrieben. Man erhält 2,8 g 10-(2'-Naphthoxy)-decylhydroxamsäure. F. 122–3 °C (Z.).

Beispiel 4

Herstellung von 2-(2'-Naphthoxy)-2-p-tolyl-acethydroxamsäure

a) 2-(2'-Naphthoxy)-2-p-tolyl-essigsäure-p-nitrophenylester

Zu einer Lösung von 6,0 g 2-(2'-Naphthoxy)-2-p-tolylessigsäure in 20 ml Pyridin setzt man portionsweise unter Rühren 6,0 g Trifluoressigsäure-p-nitrophenylester (hergestellt nach der Vorschrift von S. Yakakibara und N. Innkai, Bull. Chem. Soc. Japan 1983 (1965) ) zu. Die Mischung wird 60 Minuten bei Raumtemperatur gerührt und im Wasserstrahlpumpenvakuum bei 30 °C eingedampft.

Zum Rückstand setzt man 20 ml Wasser zu und extrahiert mit 50 ml Chloroform. Die wässerige Phase wird abgetrennt und nochmals mit 30 ml Chloroform extrahiert.

Die vereinigten Chloroformlösungen werden mit 20 ml 1 n Salzsäure, 20 ml 1 n Natriumhydrogencarbonatlösung und zweimal mit je 20 ml Wasser geschüttelt, worauf die organische Phase abgetrennt, über Magnesiumsulfat getrocknet und das Lösungsmittel im Vakuum abgezogen wird.

Den Rückstand kristallisiert man aus Methanol. Der 2-(2'-Naphthoxy)-2-p-tolyl-essigsäure-p-nitrophenylester schmilzt bei 104–5 °C.

b) 2- (2'-Naphthoxy) -2-p-tolyl -acethydroxamsäure

Zu einer Suspension von 4,2 g 2-(2'-Naphthoxy)-2-p-tolyl-essigsäure-p-nitrophenylester und 0,69 g Hydroxylaminhydrochlorid in 50 ml absolutem Chloroform gibt man bei Raumtemperatur 2,75 g Triethylamin zu. Die klare Lösung wird 90 Minuten bei Raumtemperatur gerührt; anschliessend wird das Lösungsmittel bei 30 °C im Vakuum entfernt. Der Rückstand wird mit 100 ml Ether und 5 ml 2 n Salzsäure versetzt. Man trennt die Etherlösung ab, wäscht sie zweimal mit je 40 ml Wasser, trocknet über Natriumsulfat und entfernt das Lösungsmittel im Wasserstrahlpumpenvakuum. Nach Umkristallisation aus Methanol erhält man 2,7 g 2-(2'-Naphthoxy)-2-p-tolyl-acethydroxamsäure. F. 117–8 °C (Z.).

Beispiel 5

Herstellung von 2-(2'-Naphthoxy)-2-n-propyl-acethydroxamsäure

a) 2-(2'-Naphthoxy)-2-n-propyl-acetamid

Eine Mischung von 7,5 g 2-(2'-Naphthoxy)-2-n-propyl-essigsäureethylester (Kp. 111–112 °C/0,3 mbar) und 100 ml flüssigem Ammoniak wird 4 Tage bei Raumtemperatur im Autoklaven gerührt. Der Ammoniak wird anschliessend entfernt und der verbleibende Rückstand auf einer Tonkachel getrocknet. Nach Umkristallisation aus $EtOH/H_2O$ erhält man 5,1 g 2(2'-Naphthoxy)-2-n-propylacetamid. F. 126–7 °C.

b) 2-(2'-Naphthoxy)-2-n-propyl-acethydroxamsäure

Zu einer Lösung von 1,84 g Natrium in 50 ml absolutem Methanol gibt man bei 50 °C eine Lösung von 1,82 g Hydroxylaminhydrochlorid in 30 ml absolutem Methanol. Man lässt abkühlen und trennt das abgeschiedene Natriumchlorid ab. Die Lösung wird anschliessend mit einer Lösung von 5,1 g 2-(2'-Naphthoxy)-2-n-propyl-acetamid in 50 ml Methanol versetzt und 16 Stunden unter Rückfluss gekocht. Man kühlt danach ab und engt die Lösung bei Raumtemperatur im Vakuum bis zur Trockne ein. Den Rückstand schüttelt man mit 500 ml Wasser und 100 ml Ether aus. Die wässerige Phase wird abgetrennt und mit 2 n Salzsäure angesäuert und anschliessend dreimal mit je 50 ml Ether extrahiert; die etherische Lösung wird mit Magnesiumsulfat getrocknet; das Lösungsmittel wird im Vakuum entfernt.

Es werden 4,2 g kristallin-amorphe 2-(2'-Naphthoxy)-2-n-propyl-acethydroxamsäure erhalten.

Die oben erläuterten pharmakologischen Eigenschaften wurden an Modellsystemen nachgewiesen wie aus den folgenden Beispielen hervorgeht.

Beispiel 6

Hemmung der durch Arachidonsäure induzierten Kontraktion von Meerschweinchenlungenstreifen und -tracheenringen.

Die Prüfung der Verbindungen auf antiasthmatische Aktivität erfolgte am isolierten Lungenstreifen und isolierten Tracheenringen von Meerschweinchen nach aus der Literatur bekannten Messmethoden in modifizierter Form, wie oben erwähnt. Die Messungen wurden im thermostatisierten Organbad isotonisch unter Verwendung einer Kontraktionsmessvorrichtung mit Hebelaufnehmer, Messspule, Messverstärker (induktive Messung mit Hilfe eines Hochfrequenzschwingkreises) durchgeführt. Die Begasung erfolgte mit Luft. Die Suspensionslösung hatte folgende Zusammensetzung: 39,46 g NaCl, 2,2 g KCl, 6,07 g Tris, 1,0 g $CaCl_2$, 9,9 g Glucose, 1,0 ml gesättigte $MgCl_2$-Lösung, 43 ml 1 n HCl; pH 7,4.

a) Wirkung auf den durch exogene Arachidonsäure ausgelösten Spasmus des Meerschweinchenlungenstreifens.

Der Spasmus wurde durch ansteigende Konzentrationen an Arachidonsäure (konzentrierte Lösung in Ethanol, in $N_2$-Atmosphäre gelagert) ausgelöst und kumulativ gemessen. Für die kontraktionsauslösende Wirkung der Arachidonsäure ergab sich ein $ED_{50}$-Wert im Bereich von 10 µM und $ED_{84}$-Wert im Bereich von 25 µM, abhängig vom jeweils verwendeten Lungenstreifenpräparat. Die Wirkstoffe verursachten eine hoch signifikante Rechtsverschiebung der Dosis-Wirkungs-Kurven für Arachidonsäure, sichtbar an einer deutlichen Erhöhung der $ED_{50}$- und $ED_{84}$-Werte. Der Effekt lässt sich durch folgenden Wirkungsindex beschreiben:

$$i_{50} = \frac{ED_{50}\,(\text{Wirkstoff}) - ED_{50}\,(\text{Kontrolle})}{ED_{50}\,(\text{Kontrolle})}$$

In analoger Weise wurde der Index $i_{84}$ aus den Messdaten ermittelt.

| Wirkstoff | $i_{50}$ | $i_{84}$ |
|---|---|---|
| 3-t-Butyl-4-hydroxyanisol (bekannt), 100 µM | 4,6 | 3,6 |
| Nordihydroguajaretsäure (bekannt), 50 µM | 6,3 | 6,5 |
| Salicylhydroxamsäure (bekannt), 100 µM | 1,9 | 8,5 |
| 2-(2'-Naphthoxy)-acethydroxamsäure, 100 µM | 5,8 | 7,1 |

b) Wirkung auf den durch Carbacholinduzierten Spasmus der Meerschweinchentrachea

Der Spasmus wurde durch 3,9 µM Carbachol ausgelöst. Nachträglicher Zusatz der untersuchten Wirkstoffe verursachte eine starke bis sehr starke Dilatation, die bereits bei folgenden Wirkstoffkonzentrationen deutlich nachweisbar war: 100 µM 3-t-Butyl-4-hydroxyanisol, 50 µM Nordihydroguajaretsäure, 50 µM 2(2'-Naphthoxy)-acethydroxamsäure.

c) Wirkung auf den Basaltonus von Meerschweinchenlungenstreifen

Ansteigende Konzentration der Wirkstoffe wurden zum Suspensionsmedium zugesetzt. Es trat eine Dilatation auf, die kumulativ gemessen wurde. Aus den Dosis-Wirkungs-Kurven ergaben sich folgende Parameter:

| Wirkstoff | $ED_{84}$ (µM) | $ED_{50}$ (µM) |
|---|---|---|
| 3-t-Butyl-4-hydroxyanisol | 130 | 61 |
| 2-(2'-Naphthoxy)-acethydroxamsäure | 27 | 14 |

Beispiel 7

Hemmung der Aktivität der Lipoxygenase aus Kaninchenreticulocyten

Die Prüfung der Verbindungen auf antiasthmatische, antiallergische, entzündungshemmende und antithrombotische Aktivität auf der Grundlage der Hemmung der Lipoxygenasereaktion der Arachidonsäurekaskade erfolgte an einem molekularpharmakologischen Testsystem. Die Eignung dieses Testsystems für die angegebenen Tests wurde dadurch nachgewiesen, dass viele aus der Literatur bekannte Wirkstoffe dieses Prinzips (z.B. Polyacetylenfettsäuren, Pyrazolinderivate u.a.) darauf ansprechen. Gegenüber den komplexeren Systemen bietet dieses molekulare Testsystem den Vorteil, die Wirkstoff-Rezeptor-Wechselwirkungen unabhängig von etwaigen Permeationsbarrieren und Metabolisierungen des Wirkstoffes untersuchen zu können. Hierdurch konnten die Aussagen von Beispiel 6 ergänzt und präzisiert werden. Die Lipoxygenase aus Kaninchen-Reticulocyten wurde nach dem in der Literatur beschriebenen Verfahren (siehe oben) in elektrophoretisch und immunologisch reiner Form erhalten. Die Bestimmung der Lipoxygenaseaktivität erfolgte bei 25 °C über die polarographische Messung des Sauerstoffverbrauchs mittels einer Clark-Elektrode in folgendem System: 0,1 M Kaliumphosphat pH 7,4 mit 0,2% Natriumcholat und 0,53 mM Linolsäure. Die Enzymkonzentration betrug im Messansatz 25 nM. Die zu prüfenden Verbindungen wurden in Methylglycol (frisch destilliert im Vakuum) gelöst und 15 Minuten bei der Messtemperatur in Abwesenheit von Natriumcholat und Linolsäure vorinkubiert. Die Verdünnungen der Verbindungen wurden so gewählt, dass die Endkonzentration an Methylglycol im Vorinkubationsansatz 2% nicht überstieg; unter diesen Bedingungen traten keine nennenswerten Hemmungen in den Kontrollansätzen auf. Die Enzymreaktion wurde durch Zusatz von Natriumcholat und Linolsäure gestartet. Durch Variation der Wirkstoffkonzentration wurden die Titrationskurven der Hemmung und daraus die erforderlichen Konzentrationen für eine 50%ige und eine 84%ige Hemmung ($I_{50}$- bzw. $I_{84}$-Werte) ermittelt.

Tabelle 4

Hemmung der Lipoxygenase aus Kaninchen-Reticulocyten

| Verbindung | $I_{50}$ (µM) | $I_{84}$ (µM) |
|---|---|---|
| Nordihydroguajaretsäure (bekannt) | 0,5 | 1,6 |
| 3-t-Butyl-4-hydroxyanisol (bekannt) | 160 | 600 |
| 4-Nitrocatechol (bekannt) | 4,6 | 16 |
| 5,8,11-Eicosatriinsäure (bekannt) | 1,3 | |
| Salicylhydroxamsäure (bekannt) | 20 | 60 |
| 1-Hydroxy-2-naphthylhydroxamsäure | 47 | 500 |
| 2-Hydroxy-1-naphthylhydroxamsäure | 1,6 | 5,2 |
| 2-(1'-Naphthoxy)-acethydroxamsäure | 25 | 60 |
| 2-(1'-Naphthoxy)-2-ethyl-acethydroxamsäure | 42 | 130 |
| 2-(2'-Naphthoxy)-acethydroxamsäure | 2,5 | 9 |
| 2-(2'-Naphthoxy)-2-methyl-acethydroxamsäure | 40 | 120 |
| 2-(2'-Naphthoxy)-2-ethyl-acethydroxamsäure | 32 | |
| 2-(2'-Naphthoxy)-2-i-propyl-acethydroxamsäure | 27 | |
| 2-(2'-Naphthoxy)-2-n-propyl-acethydroxamsäure | 24 | |
| 2-(2'-Naphthoxy)-2-n-butuyl-acethydroxamsäure | 19 | |
| 2-(2'-Naphthoxy)-2-n-hexyl-acethydroxamsäure | 11 | |
| 2-(2'-Naphthoxy)-2-phenyl-acethydroxamsäure | 40 | |
| 2-(2'-Naphthoxy)-2-n-octyl-acethydroxamsäure | 2,3 | 7 |
| 2-(2'-Naphthoxy)-2-n-nonyl-acethydroxamsäure | 1,5 | 4,8 |
| 3-(2'-Naphthoxy)-n-propylhydroxamsäure | 7 | 15 |
| 4-(2'-Naphthoxy)-n-butylhydroxamsäure | 4 | 7,5 |
| 5-(2'-Naphthoxy)-valerylhydroxamsäure | 3,5 | 6,5 |
| 8-(2'-Naphthoxy)-octylhydroxamsäure | 2,1 | 4,6 |

Beispiel 8

Hemmung der durch Arachidonsäure oder PAF induzierten Thrombocytenaggregation

Die Prüfung der Verbindungen auf antithrombotische und thrombolytische Aktivität erfolgte am authentischen Zellsystem des Menschen in vitro. Thrombocytenreiches Plasma aus dem Blut gesunder Spender wurde durch Zentrifugation bei einer relativen Zentrifugalbeschleunigung von 1000 erhalten. Die Messung der Thrombocytenaggregation erfolgte mittels eines Aggregometers aufgrund der diffusen Lichtstreuung bzw. der Lichtabsorption der entstehenden Zellaggregate. Das thrombocytenreiche Plasma wurde bei 37 °C 3 min mit den Wirkstoffen vorinkubiert; danach wurde die Thrombocytenaggregation durch Zusatz von entweder 0,8 mM Arachidonsäure oder

1 µM Plättchenaktivierungsfaktor (PAF-Acether) ausgelöst. Die Ansätze wurden dabei mit einer Drehzahl von 800 $min^{-1}$ gerührt. Je nach der angewandten Wirkstoffkonzentration trat entweder eine starke Verzögerung oder eine vollständige Hemmung der Thrombocytenaggregation ein.

Tabelle 5
Hemmung der durch Arachidonsäure induzierten Thrombocytenaggregation

| Verbindung | Verzögerung (µM) | 2 min Totalhemmung (µM) |
|---|---|---|
| 4-Nitroacatechol (bekannt) | 40 | 60 |
| 3-t-Butyl-4-hydroxyanisol (bekannt) | 16 | 40 |
| Salicylhydroxamsäure (bekannt) | 0,8 | 2 |
| 2-Hydroxy-1-naphthylhydroxamsäure | 6,5 | 7 |
| 2-(2'-Naphthoxy)-acethydroxamsäure | 30 | 60 |

Bei der durch PAF-Acether ausgelösten Aggregation bewirkten alle in Tabelle 5 aufgelisteten untersuchten Verbindungen in einer Konzentration von 40 µM eine Auflösung der zunächst gebildeten Zellaggregate. Gleiche Effekte wurden beobachtet, wenn in gewaschenen Thrombocytensuspensionen die Aggregation mit 16 µM Arachidonsäure ausgelöst wurde. Aus diesem Verhalten geht hervor, dass die untersuchten Lipoxygenasehemmer die Thrombocytenaggregation in ihrer irreversiblen Phase blockieren und dadurch thrombolytisch aktiv sind.

Beispiel 9
Hemmung des Carrageeninödems der Rattenpfote

Das Carrageeninödem wird in der internationalen Literatur als Modellsystem für entzündungsauslösende (prophlogistische) Vorgänge benutzt und bietet die Möglichkeit der In-vivo-Testung von Verbindungen auf entzündungshemmende (antiphlogistische) Aktivität.

2-(2'-Naphthoxy)-acethydroxamsäure wurde 10 Ratten oral in einer Dosis von 200 mg/kg bei gleichzeitiger Gabe von 0,1 ml 1%iger Carrageeninlösung pro Tier appliziert. Das Ausmass des Pfotenödems wurde nach der Applikation stündlich gemessen und mit dem der Kontrollgruppe verglichen. Dabei ergaben sich folgende Ergebnisse:

Tabelle 6
Hemmung des Carrageeninödems durch 2-(2'-Naphthoxy)-acethydroxamsäure

| Zeit (h) | Hemmung (%) |
|---|---|
| 1 | 27,3 |
| 2 | 27,3 |
| 3 | 20,2$^{+}$ |
| 4 | 35,3$^{++}$ |
| 5 | 21,4 |

$^{++}$ signifikant mit P 0,01
$^{+}$ signifikant mit P 0,05.

**Patentansprüche**

1. Hydroxamsäuren der allgemeinen Formel I,

R–CO–NH–OH (I),

in der R bedeutet:
- 2-Hydroxy-1-naphthyl,
- 1'-Naphthoxyphenylmethyl oder
- substituiertes 2-Naphthoxymethyl der Formel II

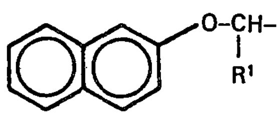
(II)

mit $R^1$ = p-Chlorphenyl oder p-Tolyl.

2. Verfahren zur Herstellung der 2-(2'-Naphthoxy)-acethydroxamsäuren nach Anspruch 1, gekennzeichnet durch an sich bekannte Umsetzung von Estern oder Amiden von Carbonsäuren der allgemeinen Formel IV

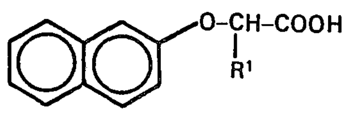
(IV)

mit $R^1$ = p-Chlorphenyl oder p-Tolyl mit Hydroxylamin oder einem Salz davon in einem basischen Lösungsmittelmedium bei einer Temperatur von 20 bis 100 °C bzw. der Siedetemperatur des Lösungsmittels und ggfs. Überführung der erhaltenen Hydroxamsäuren der Formel I mit einer anorganischen Base oder einem Metallsalz in entsprechende Salze.

3. Pharmazeutische Mittel auf der Basis von Hydroxamsäuren, gekennzeichnet durch Hydroxamsäuren der allgemeinen Formel I'

R–CO–NH–OH (I')

als Wirkstoffe, wobei R bedeutet:
- 2-Hydroxy-1-naphthyl, oder
- 1'-Naphthoxyalkyl der Formel IIa

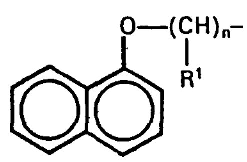
(IIa),

worin darstellen:

n 1 bis 10 und

$R^1$ für n = 1 bis 10: Wasserstoff und ausserdem für n = 1: Geradkettiges oder verzweigtes $C_{1-10}$-Alkyl oder Phenyl, oder wobei R bedeutet: 2'-Naphthoxyalkyl der Formel IIb

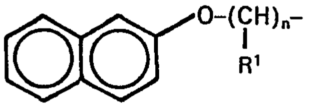

(IIb),

worin darstellen:

n 1 bis 10 und

$R^1$ für n = 1 bis 10: Wasserstoff und ausserdem für n = 1: Geradkettiges oder verzweigtes $C_{1-10}$-Alkyl, Phenyl oder substituiertes Phenyl.

4. Verwendung von Hydroxamsäuren der allgemeinen Formel I",

R–CO–NH–OH (I"),

in der R 1-Hydroxy-2-naphthyl, 2-Hydroxy-3-naphthyl oder 2-Aminophenyl bedeutet, zur Herstellung von pharmazeutischen Mitteln mit Lipoxygenase-Hemmwirkung zur Anwendung als Antiasthmatica, Antiallergica, Antiphlogistica, Bronchodilatantien, Antirheumatica und Antithrombotica.

## Revendications

1. Acides hydroxamiques répondant à la formule générale I

R–CO–NH–OH (I)

dans laquelle

R représente un groupe 2-hydroxy-1-naphtyle, 1'-naphtoxy-phénylméthyle ou un groupe 2-naphtoxyméthyle substitué répondant lui-même à la formule II

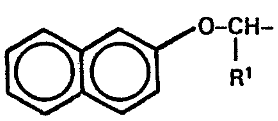

(II)

dans laquelle $R^1$ représente un groupe p-chlorophényle ou p-tolyle.

2. Procédé de préparation des acides 2-(2'-naphtoxy)acétohydroxamiques selon la revendication 1, caractérisé en ce que l'on fait réagir de manière connue en soi des esters ou amides d'acides carboxyliques de formule générale IV

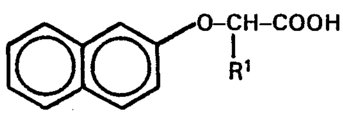

(IV)

dans laquelle $R^1$ représente un groupe p-chlorophényle ou p-tolyle, avec l'hydroxylamine ou un sel de l'hydroxylamine dans un milieu solvant basique à une température de 20 à 100 °C ou à la température d'ébullition du solvant, après quoi, le cas échéant, on convertit les acides hydroxamiques de formule I ainsi obtenus en les sels correspondants à l'aide d'une base minérale ou d'un sel métallique.

3. Produit pharmaceutique à base d'acides hydroxamiques, caractérisé en ce qu'il contient en tant que substances actives des acides hydroxamiques de formule générale I'

R–CO–NH–OH (I')

dans laquelle

R représente un groupe 2-hydroxy-1-napthyle ou un groupe 1-naphtoxyalkyle répondant lui-même à la formule IIa

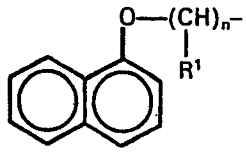

(IIa),

dans laquelle

n = 1 à 10, et

$R^1$ représente l'hydrogène lorsque n a une valeur de 1 à 10 et peut en outre représenter un groupe alkyle à chaîne droite ou ramifiée en $C_1$–$C_{10}$ ou un groupe phényle lorsque n = 1, ou bien R représente un groupe 2'-naphtoxyalkyle répondant lui-même à la formule IIb

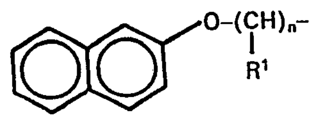

(IIb),

dans laquelle n a une valeur de 1 à 10, et

$R^1$ représente l'hydrogène lorsque n a une valeur 1 à 10 et peut en outre représenter un groupe alkyle à chaîne droite ou ramifiée en $C_1$–$C_{10}$, phényle ou phényle substitué lorsque n est égal à 1.

4. Utilisation des acides hydroxamiques de formule générale I"

R–CO–NH–OH (I")

dans laquelle R représente un groupe 1-hydroxy-2-naphtyle, 2-hydroxy-3-naphtyle ou 2-aminophényle, pour la préparation de produits pharmaceutiques ayant une activité d'inhibition de la lipoxygénase et utilisables en tant qu'anti-asthmatiques, anti-allergiques, anti-phlogistiques, bronchodilatateurs, antirhumatismaux et anti-thrombotiques.

## Claims

1. Hydroxamic acids of the general formula I,

R–CO–NH–OH (I),

wherein R stands for:

– 2-hydroxy-1-naphthyl,

– 1'-naphthoxyphenylmethyl or

– substituted 2-naphthoxymethyl of the Formula II

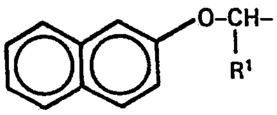

(II)

wherein $R^1$ = p-chlorophenyl or p-tolyl.

2. Process for the preparation of the 2-(2'-naphthoxy) acethydroxamic acids according to claim 1, characterized by reaction known per se of esters or amides of carboxylic acids of the general Formula IV

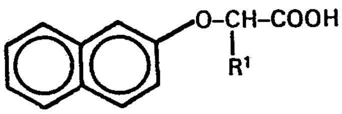

(IV)

wherein $R^1$ = p-chlorophenyl or p-tolyl with hydroxylamine or a salt thereof in a basic solvent medium at a temperature of from 20 to 100 °C respectively the boiling temperature of the solvent and, optionally, converting the obtained hydroxamic acids of the Formula I with an inorganic base or a metal salt into respective salts.

3. Pharmaceutical compositions on the basis of hydroxamic acids, characterized by hydroxamic acids of the general Formula I'

R–CO–NH–OH (I')

as active components, wherein R stands for:

- 2-hydroxy-1-naphthyl or
- 1'-naphthoxyalkyl of the Formula IIa

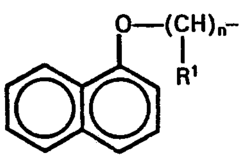

(IIa),

wherein:

n 1 to 10 and

$R^1$ for n = 1 to 10: hydrogen and furthermore for n = 1: straight-chain or branched $C_{1-10}$ alkyl or phenyl, or wherein R: 2'-naphthoxyalkyl of the Formula IIb

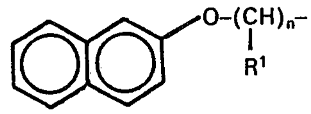

(IIb)

wherein

n 1 to 10 and

$R^1$ for n = 1 to 10: hydrogen and furthermore for n = 1: straight-chain or branched $C_{1-10}$ alkyl, phenyl or substituted phenyl.

4. The use of hydroxamic acids of the general Formula I'',

R–CO–NH–OH (I''),

wherein R 1-hydroxy -2-naphthyl, 2-hydroxy-3-naphthyl or 2-aminophenyl, for the preparation of pharmaceutical compositions with lipoxygenase inhibition activity for use as anti-asthmatics, antiallergics, antiphlogistics, bronchodilatants, antirheumatics and antithrombotics.